# EUROPEAN PATENT APPLICATION

(11) **EP 2 236 139 A1**
(43) Date of publication of application: **06.10.2010**
(21) Application number: 09004753.1
(22) Date of filing: 31.03.2009
(51) Int. Cl.: A61K 31/517, A61K 39/395, A61P 35/00

(54) **Combination therapy of erlotinib with an anti-IGF-1R antibody, which does not inhibit binding of insulin to the insulin receptor**

(71) Applicant: F.HOFFMANN-LA ROCHE AG, 4070 Basel (CH)
(72) Inventor: Juchem, Rolf, 68623 Lampertheim (DE); Kuenkele, Klaus-Peter, 83671 Benediktbeuern (DE); Wartha, Katharina, 81375 Muenchen (DE)
(74) Representative: Burger, Alexander

(57) **Abstract**

The present invention is directed to the combination treatment of cancer, especially lung cancer with an anti-IGF-1R antibody in combination with erlotinib.

## Description

The present invention is directed to the combination treatment of cancer, especially lung cancer with an anti-IGF-1R antibody in combination with erlotinib.

### Background of the Invention

Insulin-like growth factor I receptor (IGF-IR, CD 221 antigen) belongs to the family of transmembrane protein tyrosine kinases (LeRoith, D., et al., Endocrin. Rev. 16 (1995) 143-163; and Adams, T.E., et al., Cell. Mol. Life Sci. 57 (2000) 1050-1063). IGF-IR binds IGF-I with high affinity and initiates the physiological response to this ligand in vivo. IGF-IR also binds to IGF-II, however with slightly lower affinity. IGF-IR overexpression promotes the neoplastic transformation of cells and there exists evidence that IGF-IR is involved in malignant transformation of cells and is therefore a useful target for the development of therapeutic agents for the treatment of cancer (Adams, T.E., et al., Cell. Mol. Life Sci. 57 (2000) 1050-1063).

Antibodies against IGF-IR are well-known in the state of the art and investigated for their antitumor effects in vitro and in vivo (Benini, S., et al., Clin. Cancer Res. 7 (2001) 1790-1797; Scotlandi, K., et al., Cancer Gene Ther. 9 (2002) 296-307; Scotlandi, K., et al., Int. J. Cancer 101 (2002) 11-16; Brunetti, A., et al., Biochem. Biophys. Res. Commun. 165 (1989) 212-218; Prigent, S.A., et al., J. Biol. Chem. 265 (1990) 9970-9977; Li, S.L., et al., Cancer Immunol. Immunother. 49 (2000) 243-252; Pessino, A., et al., Biochem. Biophys. Res. Commun. 162 (1989) 1236-1243; Surinya, K.H., et al., J. Biol. Chem. 277 (2002) 16718-16725; Soos, M.A., et al., J. Biol. Chem., 267 (1992) 12955-12963; Soos, M.A., et al., Proc. Natl. Acad. Sci. USA 86 (1989) 5217-5221; O'Brien, R.M., et al., EMBO J. 6 (1987) 4003-4010; Taylor, R., et al., Biochem. J. 242 (1987) 123-129; Soos, M.A., et al., Biochem. J. 235 (1986) 199-208; Li, S.L., et al., Biochem. Biophys. Res. Commun. 196 (1993) 92-98; Delafontaine, P., et al., J. Mol. Cell. Cardiol. 26 (1994) 1659-1673; Kull, F.C. Jr., et al. J. Biol. Chem. 258 (1983) 6561-6566; Morgan, D.O., and Roth, R.A., Biochemistry 25 (1986) 1364-1371; Forsayeth, J.R., et al., Proc. Natl. Acad. Sci. USA 84 (1987) 3448-3451; Schaefer, E.M., et al., J. Biol. Chem. 265 (1990) 13248-13253; Gustafson, T.A., and Rutter, W.J., J. Biol. Chem. 265 (1990) 18663-18667; Hoyne, P.A., et al., FEBS Lett. 469 (2000) 57-60; Tulloch, P.A., et al., J. Struct. Biol. 125 (1999) 11-18; Rohlik, Q.T., et al., Biochem. Biophys. Res. Comm. 149 (1987) 276-281; and Kalebic, T., et al., Cancer Res. 54 (1994) 5531-5534; Adams, T. E., et al., Cell. Mol. Life Sci. 57 (2000) 1050-1063; Dricu, A., et al., Glycobiology 9 (1999) 571-579; Kanter-Lewensohn, L., et al., Melanoma Res. 8 (1998) 389-397; Li, S.L., et al., Cancer Immunol. Immunother. 49 (2000) 243-252). Antibodies against IGF-IR are also described in a lot of further publications, e.g., Arteaga, C.L., et al., Breast Cancer Res. Treatment 22 (1992) 101-106; and Hailey, J., et al., Mol. Cancer Ther. 1 (2002) 1349-1353.

In particular, the monoclonal antibody against IGF-IR called αIR3 is widely used in the investigation of studying IGF-IR mediated processes and IGF-I mediated diseases such as cancer. Alpha-IR-3 was described by Kull, F.C., J. Biol. Chem. 258 (1983) 6561-6566. In the meantime, about a hundred publications have been published dealing with the investigation and therapeutic use of αIR3 in regard to its antitumor effect, alone and together with cytostatic agents such as doxorubicin and vincristine. αIR3 is a murine monoclonal antibody which is known to inhibit IGF-I binding to IGF receptor but not IGF-II binding to IGF-IR. αIR3 stimulates at high concentrations tumor cell proliferation and IGF-IR phosphorylation (Bergmann, U., et al., Cancer Res. 55 (1995) 2007-2011; Kato, H., et al., J. Biol. Chem. 268 (1993) 2655-2661). There exist other antibodies (e.g., 1H7, Li, S.L., et al., Cancer Immunol. Immunother. 49 (2000) 243-252) which inhibit IGF-II binding to IGF-IR more potently than IGF-I binding. A summary of the state of the art of antibodies and their properties and characteristics is described by Adams, T.E., et al., Cell. Mol. Life Sci. 57 (2000) 1050-1063.

Most of the antibodies described in the state of the art are of mouse origin. Such antibodies are, as is well known in the state of the art, not useful for the therapy of human patients without further alterations like chimerization or humanization. Based on these drawbacks, human antibodies are clearly preferred as therapeutic agents in the treatment of human patients. Human antibodies are well-known in the state of the art (van Dijk, M.A., and van de Winkel, J.G., Curr. Opin. Pharmacol. 5 (2001) 368-374). Based on such technology, human antibodies against a great variety of targets can be produced. Examples of human antibodies against IGF-IR are described in WO 02/053596.

US 2005/0008642A1 describes in detail anti-IGF-1R antibodies, especially the human anti-IGF-1R antibodies <IGF-1R> HUMAB-Clone 18 (Deposition No. DSM ACC 2587) and <IGF-1R> HUMAB-Clone 22 (Deposition No. DSM ACC 2594) which a shows no detectable concentration dependent inhibition of insulin binding to the insulin receptor and are useful as therapeutic agents in the treatment of human patients.

Over-expression of the epidermal growth factor receptor (EGFR) kinase, or its ligand TGF-alpha, is frequently associated with many cancers, including breast, lung, colorectal and head and neck cancers (Salomon, D.S., et al. (1995) Crit. Rev. Oncol. Hematol. 19:183-232; Wells, A. (2000) Signal, 1:4-11), and is believed to contribute to the malignant growth of these tumors. A specific deletion-mutation in the EGFR gene has also been found to increase cellular tumorigenicity (Halatsch, M-E. et al. (2000) J. Neurosurg. 92:297-305; Archer, G.E. et al. (1999) Clin. Cancer Res. 5:2646-2652). Activation of EGFR stimulated signaling pathways promote multiple processes that are potentially cancer-promoting, e.g. proliferation, angiogenesis, cell motility and invasion, decreased apoptosis and induction of drug resistance. The development for use as anti-tumor agents of compounds that directly inhibit the kinase activity of the EGFR, as well as antibodies that reduce EGFR kinase activity by blocking EGFR activation, are areas of intense research effort (de Bono J.S. and Rowinsky, E.K. (2002) Trends in Mol. Medicine 8:S19-S26; Dancey, J. and Sausville, E.A. (2003) Nature Rev. Drug Discovery 2:92-313).

The EGFR kinase inhibitor erlotinib ([6,7-bis(2-methoxyethoxy)-4-quinazolin-4-yl]-(3-ethynylphenyl)amine, e.g. erlotinib HCL, Tarceva™) have shown promise in cancer treatment.

WO 2002/053596, WO 2005/016967, WO 2006/008639, US 2005/0249730, US 2005/0084906, WO 2005/058967, WO 2006/013472, WO 2005/016970, WO 04/083248 WO 2005/094376, WO 2005/052005 refer to anti IGF-1R antibodies and their combination inter alia with EGFR inhibitors.

### Summary of the Invention

The invention comprise the use of an antibody binding to human IGF-1R comprising as complementarity determining regions (CDRs) the following sequences:
a) in the heavy chain variable domain a CDR3 region of SEQ ID NO: 1, a CDR2 region of SEQ ID NO: 2, and a CDR1 region of SEQ ID NO:3, and in the light chain variable domain a CDR3 region of SEQ ID NO: 4, a CDR2 region of SEQ ID NO:5, and a CDR1 region of SEQ ID NO:6; or
b) in the heavy chain variable domain a CDR3 region of SEQ ID NO: 7, a CDR2 region of SEQ ID NO: 8, and a CDR1 region of SEQ ID NO:9, and in the light chain variable domain a CDR3 region of SEQ ID NO: 10, a CDR2 region of SEQ ID NO:11, and a CDR1 region of SEQ ID NO:12;
for the manufacture of a medicament for treatment of an IGF-1R and EGFR expressing cancer **characterized in that** said antibody is co-administered with erlotinib.

Such use or such combination treatment according to the invention with said anti-IGF-1R antibody (which do not inhibit binding of insulin to insulin) and erlotinib show synergistic benefits for patients in need of antitumor therapy and provide reduction of tumor growth and a significant prolongation of the time to progression. The combination treatment according to the invention have new and inventive properties causing a benefit for a patient suffering from lung cancer, especially non-small lung cancer.

The invention further comprise an antibody binding to human IGF-1R comprising as complementarity determining regions (CDRs) the following sequences:
a) in the heavy chain variable domain a CDR3 region of SEQ ID NO: 1, a CDR2 region of SEQ ID NO: 2, and a CDR1 region of SEQ ID NO:3, and in the light chain variable domain a CDR3 region of SEQ ID NO: 4, a CDR2 region of SEQ ID NO:5, and a CDR1 region of SEQ ID NO:6; or
b) in the heavy chain variable domain a CDR3 region of SEQ ID NO: 7, a CDR2 region of SEQ ID NO: 8, and a CDR1 region of SEQ ID NO:9, and in the light chain variable domain a CDR3 region of SEQ ID NO: 10, a CDR2 region of SEQ ID NO:11, and a CDR1 region of SEQ ID NO:12;
for the treatment of cancer for treatment of cancer **characterized in that** said antibody is co-administered with erlotinib

The invention further comprise a kit comprising
i) an antibody binding to human IGF-1R comprising as complementarity determining regions (CDRs) the following sequences:
   a) in the heavy chain variable domain a CDR3 region of SEQ ID NO: 1, a CDR2 region of SEQ ID NO: 2, and a CDR1 region of SEQ ID NO:3, and in the light chain variable domain a CDR3 region of SEQ ID NO: 4, a CDR2 region of SEQ ID NO:5, and a CDR1 region of SEQ ID NO:6; or
   b) in the heavy chain variable domain a CDR3 region of SEQ ID NO: 7, a CDR2 region of SEQ ID NO: 8, and a CDR1 region of SEQ ID NO:9, and in the light chain variable domain a CDR3 region of SEQ ID NO: 10, a CDR2 region of SEQ ID NO:11, and a CDR1 region of SEQ ID NO:12; and
ii) erlotinib
for the combination treatment of a patient suffering from a IGF-1R and EGFR expressing cancer.

The invention further comprise a pharmaceutical composition comprising both
i) an antibody binding to human IGF-1R comprising as complementarity determining regions (CDRs) the following sequences:
   a) in the heavy chain variable domain a CDR3 region of SEQ ID NO: 1, a CDR2 region of SEQ ID NO: 2, and a CDR1 region of SEQ ID NO:3, and in the light chain variable domain a CDR3 region of SEQ ID NO: 4, a CDR2 region of SEQ ID NO:5, and a CDR1 region of SEQ ID NO:6; or
   b) in the heavy chain variable domain a CDR3 region of SEQ ID NO: 7, a CDR2 region of SEQ ID NO: 8, and a CDR1 region of SEQ ID NO:9, and in the light chain variable domain a CDR3 region of SEQ ID NO: 10, a CDR2 region of SEQ ID NO:11, and a CDR1 region of SEQ ID NO:12; and
ii) erlotinib
for use in the treatment of an IGF-1R AND EGFR expressing cancer, wherein said pharmaceutical composition may further comprise one or more pharmaceutically acceptable carriers.

### Detailed Description of the Invention

The term "antibody" encompasses the various forms of antibodies including but not being limited to whole antibodies, antibody fragments, human antibodies, humanized antibodies and genetically engineered antibodies as long as the characteristic properties according to the invention are retained.

"Antibody fragments" comprise a portion of a full length antibody, generally at least the antigen binding portion or the variable region thereof. Examples of antibody fragments include diabodies, single-chain antibody molecules, immunotoxins, and multispecific antibodies formed from antibody fragments. In addition, antibody fragments comprise single chain polypeptides having the characteristics of a VH chain, namely being able to assemble together with a VL chain or of a VL chain binding to IGF-1R, namely being able to assemble together with a VH chain to a functional antigen binding pocket and thereby providing the property of inhibiting the binding of IGF-I and IGF-II to IGF-IR.

"Antibody fragments" also comprises such fragments which per se are not able to provide effector functions (ADCC/CDC) but provide this function in a manner according to the invention after being combined with appropriate antibody constant domain(s).

The terms "monoclonal antibody" or "monoclonal antibody composition" as used herein refer to a preparation of antibody molecules of a single amino acid composition. Accordingly, the term "human monoclonal antibody" refers to antibodies displaying a single binding specificity which have variable and constant regions derived from human germline immunoglobulin sequences. In one embodiment, the human monoclonal antibodies are produced by a hybridoma which includes a B cell obtained from a transgenic non-human animal, e.g. a transgenic mouse, having a genome comprising a human heavy chain transgene and a light human chain transgene fused to an immortalized cell.

The term "chimeric antibody" refers to a monoclonal antibody comprising a variable region, i.e., binding region, from one source or species and at least a portion of a constant region derived from a different source or species, usually prepared by recombinant DNA techniques. Chimeric antibodies comprising a murine variable region and a human constant region are especially preferred. Such murine/human chimeric antibodies are the product of expressed immunoglobulin genes comprising DNA segments encoding murine immunoglobulin variable regions and DNA segments encoding human immunoglobulin constant regions. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art. See, e.g., Morrison, S.L., et al., Proc. Natl. Acad Sci. USA 81 (1984) 6851-6855; US 5,202,238 and US 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Particularly preferred CDRs correspond to those representing sequences recognizing the antigens noted above for chimeric and bifunctional antibodies.

The term "human antibody", as used herein, is intended to include antibodies having variable and constant regions derived from human germline immunoglobulin sequences. The variable heavy chain is preferably derived from germline sequence DP-50 (GenBank LO6618) and the variable light chain is preferably derived from germline sequence L6 (GenBank X01668). The constant regions of the antibody are constant regions of human IgG1 type. Such regions can be allotypic and are described by, e.g., Johnson, G., and Wu, T.T., Nucleic Acids Res. 28 (2000) 214-218 and the databases referenced therein and are useful as long as the properties of induction of ADCC and preferably CDC according to the invention are retained.

The term "recombinant human antibody", as used herein, is intended to include all human antibodies that are prepared, expressed, created or isolated by recombinant means, such as antibodies isolated from a host cell such as an SP2-0, NSO or CHO cell or from an animal (e.g. a mouse) that is transgenic for human immunoglobulin genes or antibodies expressed using a recombinant expression vector transfected into a host cell. Such recombinant human antibodies have variable and constant regions derived from human germline immunoglobulin sequences in a rearranged form. The recombinant human antibodies according to the invention have been subjected to in vivo somatic hypermutation. Thus, the amino acid sequences of the VH and VL regions of the recombinant antibodies are sequences that, while derived from and related to human germline VH and VL sequences, may not naturally exist within the human antibody germline repertoire in vivo.

Insulin-like growth factor I receptor (SEQ ID NO:20) (IGF-IR, CD 221 antigen) belongs to the family of transmembrane protein tyrosine kinases (LeRoith, D., et al., Endocrin. Rev. 16 (1995) 143-163; and Adams, T.E., et al., Cell. Mol. Life Sci. 57 (2000) 1050-1063). IGF-IR binds IGF-I with high affinity and initiates the physiological response to this ligand in vivo. IGF-IR also binds to IGF-II, however with slightly lower affinity. IGF-IR overexpression promotes the neoplastic transformation of cells and there exists evidence that IGF-IR is involved in malignant transformation of cells and is therefore a useful target for the development of therapeutic agents for the treatment of cancer (Adams, T.E., et al., Cell. Mol. Life Sci. 57 (2000) 1050-1063).

In one aspect the invention further comprises the use of an antibody binding to human IGF-1R comprising as complementarity determining regions (CDRs) the following sequences:
a) in the heavy chain variable domain a CDR3 region of SEQ ID NO: 1, a CDR2 region of SEQ ID NO: 2, and a CDR1 region of SEQ ID NO:3, and in the light chain variable domain a CDR3 region of SEQ ID NO: 4, a CDR2 region of SEQ ID NO:5, and a CDR1 region of SEQ ID NO:6; or
b) in the heavy chain variable domain a CDR3 region of SEQ ID NO: 7, a CDR2 region of SEQ ID NO: 8, and a CDR1 region of SEQ ID NO:9, and in the light chain variable domain a CDR3 region of SEQ ID NO: 10, a CDR2 region of SEQ ID NO:11, and a CDR1 region of SEQ ID NO:12; for the manufacture of a medicament for treatment of an IGF-1R and EGFR expressing cancer **characterized in that** said antibody is co-administered with erlotinib.
Such "antibody binding to human IGF-1R" according to the invention and its preparation is described in detail US 2005/0008642A1 which is incorporated by reference.

In one embodiment such antibody binding to human IGF-1R refers to an antibody comprising as complementarity determining regions (CDRs) the following sequences:
a) in the heavy chain variable domain a CDR3 region of SEQ ID NO: 1, a CDR2 region of SEQ ID NO: 2, and a CDR1 region of SEQ ID NO:3, and in the light chain variable domain a CDR3 region of SEQ ID NO: 4, a CDR2 region of SEQ ID NO:5, and a CDR1 region of SEQ ID NO:6; or
b) in the heavy chain variable domain a CDR3 region of SEQ ID NO: 7, a CDR2 region of SEQ ID NO: 8, and a CDR1 region of SEQ ID NO:9, and in the light chain variable domain a CDR3 region of SEQ ID NO: 10, a CDR2 region of SEQ ID NO:11, and a CDR1 region of SEQ ID NO:12.

In another embodiment such antibody binding to human IGF-1R refers to an antibody comprising the following sequences:
a) as heavy chain variable domain SEQ ID NO: 13 or SEQ ID NO: 14, and
b) as light chain variable domain a SEQ ID NO: 15 or SEQ ID NO: 16.

In another embodiment such antibody binding to human IGF-1R refers to an antibody comprising the following sequences:
a) as heavy chain variable domain SEQ ID NO: 13, and
b) as light chain variable domain a SEQ ID NO: 15.

In another embodiment such antibody binding to human IGF-1R refers to an antibody comprising the following sequences:
a) as heavy chain variable domain SEQ ID NO: 14, and
b) as light chain variable domain a SEQ ID NO: 16.

In another embodiment such antibody binding to human IGF-1R refers to a human anti-IGF-1R antibodies with an amino acid sequence of <IGF-1R> HUMAB-Clone 18 (Deposition No. DSM ACC 2587) or of <IGF-1R> HUMAB-Clone 22 (Deposition No. DSM ACC 2594) , preferably of <IGF-1R> HUMAB-Clone 18 (Deposition No. DSM ACC 2587).

The antibody shows preferably no detectable concentration dependent inhibition of insulin binding to the insulin receptor. The antibodies according to the invention preferably do not inhibit binding of insulin to insulin receptor in a binding competition assay on insulin receptor overexpressing 3T3 cells using the antibody in a concentration of 200 nmol/l.

The term "nucleic acid molecule", as used herein, is intended to include DNA molecules and RNA molecules. A nucleic acid molecule may be single-stranded or double-stranded, but preferably is double-stranded DNA.

The "constant domains" are not involved directly in binding the antibody to an antigen but are involved in the effector functions (ADCC, complement binding, and CDC). The constant domain of an antibody according to the invention is of the IgG1 type. Human constant domains having these characteristics are described in detail by Kabat et al., Sequences of Proteins of Immunological Interest, 5th Ed. Public Health Service, National Institutes of Health, Bethesda, MD. (1991), and by Brüggemann, M., et al., J. Exp. Med. 166 (1987) 1351-1361; Love, T.W., et al., Methods Enzymol. 178 (1989) 515-527. Examples are shown in SEQ ID NOS:5 to 8. Other useful and preferred constant domains are the constant domains of the antibodies obtainable from the hybridoma cell lines deposited with DSMZ for this invention. The constant domains useful in the invention provide complement binding. ADCC and optionally CDC are provided by the combination of variable and constant domains.

The "variable region" (variable region of a light chain (VL), variable region of a heavy chain (VH)) as used herein denotes each of the pair of light and heavy chains which is involved directly in binding the antibody to the antigen. The domains of variable human light and heavy chains have the same general structure and each domain comprises four framework (FR) regions whose sequences are widely conserved, connected by three "hypervariable regions" (or complementarity determining regions, CDRs). The framework regions adopt a β-sheet conformation and the CDRs may form loops connecting the β-sheet structure. The CDRs in each chain are held in their three-dimensional structure by the framework regions and form together with the CDRs from the other chain the antigen binding site. The antibody heavy and light chain CDR3 regions play a particularly important role in the binding specificity/affinity of the antibodies according to the invention and therefore provide a further object of the invention.

The terms "hypervariable region" or "antigen-binding portion of an antibody" when used herein refer to the amino acid residues of an antibody which are responsible for antigen-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. Especially, CDR3 of the heavy chain is the region which contributes most to antigen binding. CDR and FR regions are determined according to the standard definition of Kabat et al., Sequences of Proteins of Immunological Interest, 5th Ed. Public Health Service, National Institutes of Health, Bethesda, MD. (1991)) and/or those residues from a "hypervariable loop".

The term "binding to human IGF-IR" as used herein means the binding of the antibody to IGF-IR in an in vitro assay, preferably in a binding assay in which the antibody is bound to a surface and binding of IGF-IR is measured by Surface Plasmon Resonance (SPR). Binding means a binding affinity (K_{D}) of 10⁻⁸ M or less, preferably 10⁻¹³ to 10⁻⁹ M. Binding to IGF-IR can be investigated by a BIAcore assay (Pharmacia Biosensor AB, Uppsala, Sweden). The affinity of the binding is defined by the terms ka (rate constant for the association of the antibody from the antibody/antigen complex), kd (dissociation constant), and K_{D} (kd/ka). Thus the antibody binding to human IGF-1R according to the invention has a a binding affinity (K_{D}) of 10⁻⁸ M or less, preferably of 10⁻¹³ to 10⁻⁹ M.

The binding of IGF-I and IGF-II to IGF-IR is also inhibited by the antibodies according to the invention. The inhibition is measured as IC₅₀ in an assay for binding of IGF-I/IGF-II to IGF-IR on tumor cells. Such an assay is described in Example 7. In such an assay, the amount of radiolabeled IGF-I or IGF-II or IGF-IR binding fragments thereof bound to the IGF-IR provided at the surface of said tumor cells (e.g. HT29) is measured without and with increasing concentrations of the antibody. The IC₅₀ values of the antibodies according to the invention for the binding of IGF-I and IGF-II to IGF-IR are no more than 2 nM and the ratio of the IC₅₀ values for binding of IGF-I/IGF-II to IGF-IR is about 1:3 to 3:1. IC₅₀ values are measured as average or median values of at least three independent measurements. Single IC₅₀ values may be out of the scope.

The term "antibody-dependent cellular cytotoxicity (ADCC)" refers to lysis of human tumor target cells by an antibody according to the invention in the presence of effector cells. ADCC is measured preferably by the treatment of a preparation of IGF-IR expressing cells with an antibody according to the invention in the presence of effector cells such as freshly isolated PBMC or purified effector cells from buffy coats, like monocytes or NK cells. ADCC is found if the antibody induces at a concentration of 100 nM the lysis (cell death) of 20% or more of the tumor cells after 24 hours. If ADCC is found to be more pronounced at 4 h than at 24 h, then measurement is performed at 4 h. The assay is performed preferably with ⁵¹Cr or Eu labeled tumor cells and measurement of specifically released ⁵¹Cr or Eu. Controls include the incubation of the tumor target cells with effector cells but without the antibody.

The term "complement-dependent cytotoxicity (CDC)" refers to lysis of human tumor target cells by the antibody according to the invention in the presence of complement. CDC is measured preferably by the treatment of a preparation of IGF-IR expressing cells with an antibody according to the invention in the presence of complement. CDC is found if the antibody induces at a concentration of 100 nM the lysis (cell death) of 20% or more of the tumor cells after 4 hours. The assay is performed preferably with ⁵¹Cr or Eu labeled tumor cells and measurement of released ⁵¹Cr or Eu. Controls include the incubation of the tumor target cells with complement but without the antibody.

The antibodies binding to human IGF-1R according to the invention show a binding to the same epitope of IGF-1R as <IGF-1R> HUMAB-Clone 18 or are inhibited in binding to IGF-IR due to steric hindrance of binding by <IGF-1R> HUMAB-Clone 18. Binding inhibition can be detected by an SPR assay using immobilized <IGF-1R> HUMAB-Clone 18 and IGF-IR at a concentration of 20-50 nM and the antibody to be detected at a concentration of 100 nM. A signal reduction of 50 % or more shows that the antibody competes with antibody 18. Such an assay can be performed in the same manner by using antibody 22 as an immobilized antibody.

The term "epitope" means a protein determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Human epidermal growth factor receptor (also known as HER-1 or Erb-B1, and referred to herein as "EGFR") is a 170 kDa transmembrane receptor encoded by the c-erbB proto-oncogene, and exhibits intrinsic tyrosine kinase activity (Modjtahedi, H., et al., Br. J. Cancer 73:228-235 (1996); Herbst and Shin, Cancer 94:1593-1611 (2002)). (SEQ ID NO:21) There are also isoforms and variants of EGFR (e.g., alternative RNA transcripts, truncated versions, polymorphisms, etc.) including but not limited to those identified by Swissprot database entry numbers P00533-1, P00533-2, P00533-3, and P00533-4. EGFR is known to bind ligands including epidermal growth factor (EGF), transforming growth factor-α (TGf-α), amphiregulin, heparin-binding EGF (hb-EGF), betacellulin, and epiregulin (Herbst and Shin, Cancer 94:1593-1611 (2002); Mendelsohn and Baselga, Oncogene 19:6550-6565 (2000)). EGFR regulates numerous cellular processes via tyrosine-kinase mediated signal transduction pathways, including, but not limited to, activation of signal transduction pathways that control cell proliferation, differentiation, cell survival, apoptosis, angiogenesis, mitogenesis, and metastasis (Atalay, G., et al., Ann. Oncology 14:1346-1363 (2003); Tsao and Herbst, Signal 4:4-9 (2003); Herbst and Shin, Cancer 94:1593-1611 (2002); Modjtahedi, H., et al., Br. J. Cancer 73:228-235 (1996)).

The EGFR kinase inhibitor "erlotinib" is [6,7-bis(2-methoxyethoxy)-4-quinazolin-4-yl]-(3-ethynylphenyl)amine, also known as OSI-774; or as erlotinib HCl, registered under the trade name Tarceva™(from OSI Pharmaceuticals/Genentech/Roche).The preparation and use of erlotinib is described e.g. in detail in US 5,747,498; WO 01/34574, and Moyer, J.D. et al. (1997) Cancer Res. 57:4838-4848.

The terms "expression of IGF-1R and EGFR" antigen or "IGF-1R and EGFR expressing" are intended to indicate an significant level of expression of the IGF-1R and EGFR antigen in a cancer cell.

Patients having a "IGF-1R and EGFR expressing cancer" can be determined by standard assays known in the art. E.g. IGF-1R and EGFR antigen expression is measured using immunohistochemical (IHC) detection, FACS or via PCR-based detection of the corresponding mRNA. Preferably expression is measured in fixed cells of frozen or paraffin-embedded tissue sections using immunohistochemical (IHC) detection. When coupled with histological staining, localization of the targeted protein can be determined and extent of its expression within a tumor can be measured both qualitatively and semi-quantitatively. Such IHC detection assays are known in the art and include the Clinical Trial Assay (CTA). The latter assay uses a specific range of 0 to 3+ cell staining (0 being normal expression, 3+ indicating the strongest positive expression) to identify cancers having overexpression of the antigen. Thus, patients having a cancer characterized by expression of IGF-1R and EGFR protein in the range of 1+, 2+, or 3+, preferably 2+ or 3+, more preferably 3+ for both the IGF-1R and EGFR protein would benefit from the methods of therapy of the present invention. Human cancer cells which express IGF-1R and EGFR protein in the range of 2+, or 3+ are e.g. the lung cancer cell lines H322M (ATCC Catalogue No.); A549(ATCC Catalogue No. CCL-185), QG56, the colorectal cancer cell line HT-29 (ATCC Catalogue No. HTB-38), the breast cancer cell line KPL4, the pancreatic cancer cell lines Panc-1 (ATCC Catalogue No. CRL-1469) and (ATCC Catalogue No.) ovarian cancer cell line SKOV3 (ATCC Catalogue No. HTB-77)and the skin/epidermal cancer cell line A431 (ATCC Catalogue No. CRL-1555).

The term "IGF-1R and EGFR expressing cancer" as used herein refers to all cancers in which the cancer cells show an expression of IGF-1R and EGFR antigen. Such IGF-1R and EGFR expressing cancers may be, for example, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or urethra, renal cell carcinoma, carcinoma of the renal pelvis, mesothelioma, hepatocellular cancer, biliary cancer, chronic or acute leukemia, lymphocytic lymphomas, neoplasms of the central nervous system (CNS), epidermal/skin cancer, spinal axis tumors, brain stem glioma, glioblastoma multiforme, astrocytomas, schwannomas, ependymomas, medulloblastomas, meningiomas, squamous cell carcinomas, pituitary adenomas, including refractory versions of any of the above cancers, or a combination of one or more of the above cancers. The precancerous condition or lesion includes, for example, the group consisting of oral leukoplakia, actinic keratosis (solar keratosis), precancerous polyps of the colon or rectum, gastric epithelial dysplasia, adenomatous dysplasia, hereditary nonpolyposis colon cancer syndrome (HNPCC), Barrett's esophagus, bladder dysplasia, and precancerous cervical conditions. In a preferred embodiment, the cancer is a lung cancer, and preferably non small cell lung (NSCL) cancer. In antoher preferred embodiment the cancer is lung cancer, colorectal cancer , breast cancer, pancreatic cancer , ovarian cancer or epidermal cancer.

The term "a method of treating" or its equivalent, when applied to, for example, cancer refers to a procedure or course of action that is designed to reduce or eliminate the number of cancer cells in a patient, or to alleviate the symptoms of a cancer. "A method of treating" cancer or another proliferative disorder does not necessarily mean that the cancer cells or other disorder will, in fact, be eliminated, that the number of cells or disorder will, in fact, be reduced, or that the symptoms of a cancer or other disorder will, in fact, be alleviated. Often, a method of treating cancer will be performed even with a low likelihood of success, but which, given the medical history and estimated survival expectancy of a patient, is nevertheless deemed to induce an overall beneficial course of action.

The terms "co-administration" or "co-administering " refer to the administration of said anti-IGF-1R antibody and erlotinib preferably as two separate formulations (or eventually as one single formulation). The co-administration can be simultaneous or sequential in either order, wherein preferably there is a time period while both (or all) active agents simultaneously exert their biological activities. Said anti-IGF-1 R antibody and erlotinib are co-administered either simultaneously or sequentially (e.g. via an intravenous (i.v.) through a continuous infusion (one for the antibody and eventually one for erlotinib). When both therapeutic agents are co-administered sequentially the dose is administered either on the same day in two separate administrations, or one of the agents is administered on day 1 and the second is co-administered on day 2 to day 7, preferably on day 2 to 4. Thus the term "sequentially" means within 7 days after the dose of the first antibody, preferably within 4 days after the dose of the first antibody; and the term "simultaneously" means at the same time. The terms "co-administration" with respect to the maintenance doses of said anti-IGF-1R antibody and erlotinib mean that the maintenance doses can be either co-administered simultaneously, if the treatment cycle is appropriate for both drugs, e.g. every week. Or erlotinib is e.g. administered e.g. every first to third day and said anti-IGF-1R antibody is administered every week. Or the maintenance doses are co-administered sequentially, either within one or within several days.

It is self-evident that the antibodies are administered to the patient in a "therapeutically effective amount" (or simply "effective amount") which is the amount of the respective compound or combination that will elicit the biological or medical response of a tissue, system, animal or human that is being sought by the researcher, veterinarian, medical doctor or other clinician.

The amount of co-administration of said anti-IGF-1R antibody and erlotinib and the timing of co-administration will depend on the type (species, gender, age, weight, etc.) and condition of the patient being treated and the severity of the disease or condition being treated. Said anti-IGF-1R antibody and erlotinib are suitably co-administered to the patient at one time or over a series of treatments. Depending on the type and severity of the disease, about 1 µg /kg to 50 mg/kg (e.g. 0.1-20 mg/kg) of said anti-IGF-1R antibody and from 5-200 mg of erlotinib per day is an initial candidate dosage for co-administration of both drugs to the patient. If the administration is intravenous the initial infusion time for said anti-IGF-1R antibody or erlotinib may be longer than subsequent infusion times, for instance approximately 90 minutes for the initial infusion, and approximately 30 minutes for subsequent infusions (if the initial infusion is well tolerated).

The preferred dosage of said anti-IGF-1R antibody will be in the range from about 0.05mg/kg to about 30mg/kg. Thus, one or more doses of about 0.5mg/kg, 2.0mg/kg, 4.0mg/kg, 10mg/kg or 30mg/kg (or any combination thereof) may be co-administered to the patient. The preferred dosage of erlotinib will be in the range from 5-200 mg per day, or 100-1600 mg per week, in single or divided doses, or by continuous infusion. A preferred dose is 150 mg/day.

In a preferred embodiment, the medicament is useful for preventing or reducing metastasis or further dissemination in such a patient suffering from IGF-1R and EGFR expressing cancer. The medicament is useful for increasing the duration of survival of such a patient, increasing the progression free survival of such a patient, increasing the duration of response, resulting in a statistically significant and clinically meaningful improvement of the treated patient as measured by the duration of survival, progression free survival, response rate or duration of response. In a preferred embodiment, the medicament is useful for increasing the response rate in a group of patients.

In the context of this invention, additional cytotoxic, chemotherapeutic or anti-cancer agents, or compounds that enhance the effects of such agents (e.g. cytokines) may be used in the anti-IGF-1R antibody and erlotinib combination treatment of IGF-1R and EGFR expressing cancer. Such molecules are suitably present in combination in amounts that are effective for the purpose intended. Preferably said anti-IGF-1R antibody and erlotinib combination treatment is used without such additional cytotoxic, chemotherapeutic or anti-cancer agents, or compounds that enhance the effects of such agents.

Such agents include, for example: alkylating agents or agents with an alkylating action, such as cyclophosphamide (CTX; e.g. cytoxan®), chlorambucil (CHL; e.g. leukeran®), cisplatin (CisP; e.g. platinol®) busulfan (e.g. myleran®), melphalan, carmustine (BCNU), streptozotocin, triethylenemelamine (TEM), mitomycin C, and the like; anti-metabolites, such as methotrexate (MTX), etoposide (VP16; e.g. vepesid®), 6-mercaptopurine (6MP), 6-thiocguanine (6TG), cytarabine (Ara-C), 5-fluorouracil (5-FU), capecitabine (e.g. Xeloda®), dacarbazine (DTIC), and the like; antibiotics, such as actinomycin D, doxorubicin (DXR; e.g. adriamycin®), daunorubicin (daunomycin), bleomycin, mithramycin and the like; alkaloids, such as vinca alkaloids such as vincristine (VCR), vinblastine, and the like; and other antitumor agents, such as paclitaxel (e.g. taxol®) and paclitaxel derivatives, the cytostatic agents, glucocorticoids such as dexamethasone (DEX; e.g. decadron®) and corticosteroids such as prednisone, nucleoside enzyme inhibitors such as hydroxyurea, amino acid depleting enzymes such as asparaginase, leucovorin and other folic acid derivatives, and similar, diverse antitumor agents. The following agents may also be used as additional agents: arnifostine (e.g. ethyol®), dactinomycin, mechlorethamine (nitrogen mustard), streptozocin, cyclophosphamide, lomustine (CCNU), doxorubicin lipo (e.g. doxil®), gemcitabine (e.g. gemzar®), daunorubicin lipo (e.g. daunoxome®), procarbazine, mitomycin, docetaxel (e.g. taxotere®), aldesleukin, carboplatin, oxaliplatin, cladribine, camptothecin, CPT 11 (irinotecan), 10-hydroxy 7-ethyl-camptothecin (SN38), floxuridine, fludarabine, ifosfamide, idarubicin, mesna, interferon beta, interferon alpha, mitoxantrone, topotecan, leuprolide, megestrol, melphalan, mercaptopurine, plicamycin, mitotane, pegaspargase, pentostatin, pipobroman, plicamycin, tamoxifen, teniposide, testolactone, thioguanine, thiotepa, uracil mustard, vinorelbine, chlorambucil. Preferably said anti-IGF-1R antibody and erlotinib combination treatment is used without such additional agents.

The use of the cytotoxic and anticancer agents described above as well as antiproliferative target-specific anticancer drugs like protein kinase inhibitors in chemotherapeutic regimens is generally well characterized in the cancer therapy arts, and their use herein falls under the same considerations for monitoring tolerance and effectiveness and for controlling administration routes and dosages, with some adjustments. For example, the actual dosages of the cytotoxic agents may vary depending upon the patient's cultured cell response determined by using histoculture methods. Generally, the dosage will be reduced compared to the amount used in the absence of additional other agents.

Typical dosages of an effective cytotoxic agent can be in the ranges recommended by the manufacturer, and where indicated by in vitro responses or responses in animal models, can be reduced by up to about one order of magnitude concentration or amount. Thus, the actual dosage will depend upon the judgment of the physician, the condition of the patient, and the effectiveness of the therapeutic method based on the in vitro responsiveness of the primary cultured malignant cells or histocultured tissue sample, or the responses observed in the appropriate animal models.

In the context of this invention, an effective amount of ionizing radiation may be carried out and/or a radiopharmaceutical may be used in addition to said anti-IGF-1R antibody and erlotinib combination treatment of IGF-1R and EGFR expressing cancer. The source of radiation can be either external or internal to the patient being treated. When the source is external to the patient, the therapy is known as external beam radiation therapy (EBRT). When the source of radiation is internal to the patient, the treatment is called brachytherapy (BT). Radioactive atoms for use in the context of this invention can be selected from the group including, but not limited to, radium, cesium-137, iridium-192, americium-241, gold-198, cobalt-57, copper-67, technetium-99, iodine-123, iodine-131, and indium-111. Is also possible to label the antibody with such radioactive isotopes. Preferably said anti-IGF-1R antibody and erlotinib combination treatment is used without such ionizing radiation.

Radiation therapy is a standard treatment for controlling unresectable or inoperable tumors and/or tumor metastases. Improved results have been seen when radiation therapy has been combined with chemotherapy. Radiation therapy is based on the principle that high-dose radiation delivered to a target area will result in the death of reproductive cells in both tumor and normal tissues. The radiation dosage regimen is generally defined in terms of radiation absorbed dose (Gy), time and fractionation, and must be carefully defined by the oncologist. The amount of radiation a patient receives will depend on various considerations, but the two most important are the location of the tumor in relation to other critical structures or organs of the body, and the extent to which the tumor has spread. A typical course of treatment for a patient undergoing radiation therapy will be a treatment schedule over a 1 to 6 week period, with a total dose of between 10 and 80 Gy administered to the patient in a single daily fraction of about 1.8 to 2.0 Gy, 5 days a week. In a preferred embodiment of this invention there is synergy when tumors in human patients are treated with the combination treatment of the invention and radiation. In other words, the inhibition of tumor growth by means of the agents comprising the combination of the invention is enhanced when combined with radiation, optionally with additional chemotherapeutic or anticancer agents. Parameters of adjuvant radiation therapies are, for example, contained in WO 99/60023.

Said anti-IGF-1R antibody and erlotinib are administered to a patient according to known methods, by intravenous administration as a bolus or by continuous infusion over a period of time, by intramuscular, intraperitoneal, intracerobrospinal, subcutaneous, intra-articular, intrasynovial, or intrathecal routes. Intravenous or subcutaneous administration of the antibodies is preferred.

The invention further comprises a kit comprising said anti-IGF-1R antibody and erlotinib for the combination treatment of a patient suffering from a IGF-1R and EGFR expressing cancer. In a preferred embodiment, the kit containers may further include a pharmaceutically acceptable carrier. The kit may further include a sterile diluent, which is preferably stored in a separate additional container. The kit may further include a package insert comprising printed instructions directing the use of the combined treatment as a method for a IGF-1R and EGFR expressing cancer disease, preferably a lung cancer, and preferably a non small cell lung (NSCL) cancer.

The term "package insert" refers to instructions customarily included in commercial packages of therapeutic products, which may include information about the indications, usage, dosage, administration, contraindications and/or warnings concerning the use of such therapeutic products.

In a preferred embodiment, the article of manufacture containers may further include a pharmaceutically acceptable carrier. The article of manufacture may further include a sterile diluent, which is preferably stored in a separate additional container.

As used herein, a "pharmaceutically acceptable carrier" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, use thereof in the compositions of the invention is contemplated. Supplementary active compounds can also be incorporated into the compositions.

Pharmaceutical Compositions:
Pharmaceutical compositions can be obtained by processing said anti-IGF-1R antibody and/or and erlotinib according to this invention with pharmaceutically acceptable, inorganic or organic carriers. Lactose, corn starch or derivatives thereof, talc, stearic acids or it's salts and the like can be used, for example, as such carriers for tablets, coated tablets, dragées and hard gelatine capsules. Suitable carriers for soft gelatine capsules are, for example, vegetable oils, waxes, fats, semi-solid and liquid polyols and the like. Depending on the nature of the active substance no carriers are, however, usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical compositions can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

One embodiment of the invention is pharmaceutical composition comprising both said anti-IGF-1R antibody and erlotinib, in particular for use in IGF-1R and EGFR expressing cancer.

Said pharmaceutical composition may further comprise one or more pharmaceutically acceptable carriers.

The present invention further provides a pharmaceutical composition, in particular for use in cancer, comprising (i) an effective first amount of said anti-IGF-1R antibody, and (ii) an effective second amount of erlotinib. Such composition optionally comprises pharmaceutically acceptable carriers and / or excipients.

Pharmaceutical compositions of said anti-IGF-1R antibody alone used in accordance with the present invention are prepared for storage by mixing an antibody having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN^{™}, PLURONICS^{™} or polyethylene glycol (PEG).

Pharmaceutical compositions of erlotinib, preferably of erlotinib HCl (also known as Tarceva™) can contain various pharmaceutically acceptable inert carriers in the form of tablets, capsules, lozenges, troches, hard candies, powders, sprays, creams, salves, suppositories, jellies, gels, pastes, lotions, ointments, elixirs, syrups, and the like. Administration of such dosage forms can be carried out in single or multiple doses. Carriers include solid diluents or fillers, sterile aqueous media and various non-toxic organic solvents, etc. Oral pharmaceutical compositions can be suitably sweetened and/or flavored.

Methods of preparing pharmaceutical compositions comprising erlotinib are known in the art, and are described, e.g. in International Patent Publication No. WO 01/34574.

For oral administration of erlotinib, tablets containing one or both of the active agents are combined with any of various excipients such as, for example, microcrystalline cellulose, sodium citrate, calcium carbonate, dicalcium phosphate and glycine, along with various disintegrants such as starch (and preferably corn, potato or tapioca starch), alginic acid and certain complex silicates, together with granulation binders like polyvinyl pyrrolidone, sucrose, gelatin and acacia. Additionally, lubricating agents such as magnesium stearate, sodium lauryl sulfate and talc are often very useful for tableting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the EGFR kinase inhibitor may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration of either or both of the active agents, solutions in either sesame or peanut oil or in aqueous propylene glycol may be employed, as well as sterile aqueous solutions comprising the active agent or a corresponding water-soluble salt thereof. Such sterile aqueous solutions are preferably suitably buffered, and are also preferably rendered isotonic, e.g., with sufficient saline or glucose. These particular aqueous solutions are especially suitable for intravenous, intramuscular, subcutaneous and intraperitoneal injection purposes. The oily solutions are suitable for intra-articular, intramuscular and subcutaneous injection purposes. The preparation of all these solutions under sterile conditions is readily accomplished by standard pharmaceutical techniques well known to those skilled in the art. Any parenteral formulation selected for administration of proteinaceous EGFR kinase inhibitors should be selected so as to avoid denaturation and loss of biological activity of the inhibitor.

The active ingredients may also be entrapped in microcapsules prepared, for example, by coacervation techniques or by interracial polymerization, for example, hydroxymethylcellulose or gelatin-microcapsules and poly- (methylmethacylate) microcapsules, respectively, in colloidal drug delivery systems (for example, liposomes, albumin microspheres, microemulsions, nano- particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (US 3,773,919), copolymers of L-glutamic acid and gamma-ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes.

The present invention further provides a method for the treatment of cancer, comprising administering to a patient in need of such treatment (i) an effective first amount of said anti-IGF-1R antibody according to the invention; and (ii) an effective second amount of erlotinib.

The present invention further provides a method for the treatment of cancer, comprising administering to a patient in need of such treatment (i) an effective first amount of said anti-IGF-1R antibody according to the invention; and (ii) an effective second amount of erlotinib.

As used herein, the term "patient" preferably refers to a human in need of treatment said anti-IGF-1R antibody according to the invention and erlotinib (e.g. a patient suffering from IGF-1R and EGFR expressing cancer) for any purpose, and more preferably a human in need of such a treatment to treat cancer, or a precancerous condition or lesion. However, the term "patient" can also refer to non-human animals, preferably mammals such as dogs, cats, horses, cows, pigs, sheep and non-human primates, among others.

The invention further comprises said anti-IGF-1R antibody according to the invention and erlotinib for the treatment of a patient suffering from a IGF-1R and EGFR expressing cancer **characterized in that** said antibody is co-administered with erlotinib.

The following examples and figures are provided to aid the understanding of the present invention, the true scope of which is set forth in the appended claims. It is understood that modifications can be made in the procedures set forth without departing from the spirit of the invention.

### Sequence Listing

| | |
|---|---|
| SEQ ID NO: 1 -16 | amino acid sequences of light and heavy variable region domains of <IGF-1R> HUMAB Clone 18 (antibody 18) and <IGF-1R> HUMAB Clone 22 (antibody 22) (US 2005/0008642A1) |
| SEQ ID NO: 1 | heavy chain CDR3, <IGF-1R> HUMAB-Clone 18 |
| SEQ ID NO: 2 | heavy chain CDR2, <IGF-1R> HUMAB-Clone 18 |
| SEQ ID NO: 3 | heavy chain CDR1, <IGF-1R> HUMAB-Clone 18 |
| SEQ ID NO: 4 | light chain CDR3, <IGF-1R> HUMAB-Clone 18 |
| SEQ ID NO: 5 | light chain CDR2, <IGF-1R> HUMAB-Clone 18 |
| SEQ ID NO: 6 | light chain CDR1, <IGF-1R> HUMAB-Clone 18 |
| SEQ ID NO: 7 | heavy chain CDR3, <IGF-1R> HUMAB-Clone 22 |
| SEQ ID NO: 8 | heavy chain CDR2, <IGF-1R> HUMAB-Clone 22 |
| SEQ ID NO: 9 | heavy chain CDR1, <IGF-1R> HUMAB-Clone 22 |
| SEQ ID NO: 10 | light chain CDR3, <IGF-1R> HUMAB-Clone 22 |
| SEQ ID NO: 11 | light chain CDR2, <IGF-1R> HUMAB-Clone 22 |
| SEQ ID NO: 12 | light chain CDR1, <IGF-1R> HUMAB-Clone 22 |
| SEQ ID NO: 13 | heavy chain variable domain, <IGF-1R> HUMAB-Clone 18 |
| SEQ ID NO: 14 | heavy chain variable domain, <IGF-1R> HUMAB-Clone 22 |
| SEQ ID NO: 15 | light chain variable domain, <IGF-1R> HUMAB-Clone 18 |
| SEQ ID NO: 16 | light chain variable domain, <IGF-1R> HUMAB-Clone 22 |
| SEQ ID NO: 17 | human heavy chain constant region derived from IgG1 |
| SEQ ID NO: 18 | human heavy chain constant region derived from IgG4 |
| SEQ ID NO: 19 | kappa light chain constant region |
| SEQ ID NO: 20 | sequence of IGF-1R |
| SEQ ID NO: 21 | sequence of EGFR |

### Description of the Figures

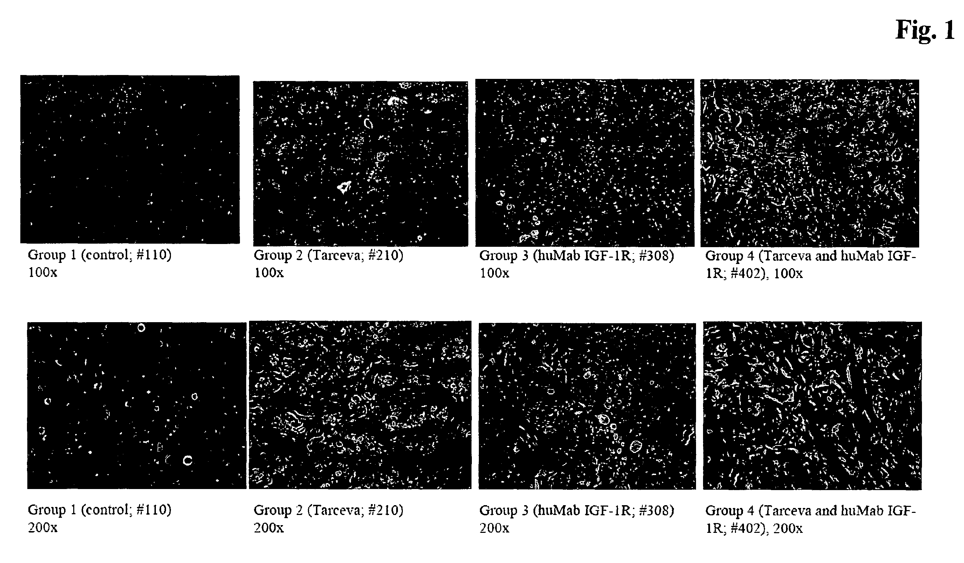

- **Figure 1**: Photographs of H322M tumors of groups 1, 2, 3 and 4 (100x and 200x magnification)-Changes in the histological properties of H322M human non small cell lung cancer tumors xenografted infemale SCID beige mice after single agent treatment with erlotinib HCl (Tarceva^{™}), or after single agent treatment with the human monoclonal antibody <IGF-1R> HUMAB-Clone 18 (antibody 18) as well as after a combination treatment with both compounds

### Experimental Procedures

### Examples 1

To investigate changes in the histological properties of H322M human non small cell lung cancer tumors xenografted infemale SCID beige mice after single agent treatment with erlotinib HCl (Tarceva^{™}), or after single agent treatment with the human monoclonal antibody <IGF-1R> HUMAB-Clone 18 (antibody 18 = MAK <IGF-1R>) directed against IGF-1R (see US 2005/0008642A1) as well as after a combination treatment with both compounds the following experiments were conducted.

### 1.1 Groups (Table 1)

4 experimental groups of female SCID beige mice were used in this study. Ten animals of each group were examined histologically. Groups and dosages can be seen in the following table 1.

**Table 1:**

| **Group** | **No of animals** | **Compound** | **Dose (mg/kg)** | **Route/ Mode of administration** | **No of treatments** | **Cumulative dose (mg/kg)** |
|---|---|---|---|---|---|---|
| 1 | 15 | Captisol | - | p.o. | 31 | 0 |
| | | 6% NaCl | | i.p. | 5 | |
| | | 0.9% | | | | |
| 2 | 15 | Tarceva | 50 | p.o. | 31 | 1550 |
| 3 | 15 | MAK<IGF- | LD 36 | i.p. | 5 | 108 |
| | | 1R> | 18 | | | |
| 4 | 15 | Tarceva + | 50 | p.o. | 31 | 1550 |
| | | MAK<IGF-1R> | LD 36 18 | i.p. | 5 | 108 |

### 1.2 Criteria for the histologic evaluation

### Rate of proliferation / Rate of apoptosis (x200 magnification per field of view)

| | | | |
|---|---|---|---|
| (+) | = | 1 | mitotic figures / apoptosis per field of view |
| + | = | 2-3 | mitotic figures / apoptosis per field of view |
| ++ | = | 4-5 | mitotic figures / apoptosis per field of view |
| +++ | = | 6-9 | mitotic figures / apoptosis per field of view |
| ++++ | = | >10 | mitotic figures / apoptosis per field of view |

### Invasive Growth

| | | |
|---|---|---|
| (+) | = | small tumor; encapsulated; mild infiltration tendency |
| + | = | tumor encapsulated; individual tumor cell in capsule |
| ++ | = | tumor encapsulated; distinct local invasion |
| ++ -+++ | = | tumor encapsulated; local invasion; suspected vascular penetration |
| +++ | = | local invasion; distinct vascular penetration |

| | | |
|---|---|---|
| Necrosis | = | estimated in % of total tumor area |
| Cellular/nuclear polymorphism | = | estimated |
| Capsule formation | = | estimated |
| Intratumoral fibrosis | = | estimated |
| Inflammatory cell infiltration | = | estimated |

### 3. RESULTS

### 3.1 Histopathological analysis of primary tumors

### Group 1: Vehicle control (Captisol 6% and NaCl 0,9%)

The tumors examined represented a poorly differentiated solid adenocarcinoma of the human non small lung cancer cell line H322M. The tumors showed a moderate to severe cellular and nuclear polymorphism sometimes with differentiation into squamous epithelium or anaplastic areas. The tumors revealed a slight to moderate capsule formation. Intratumoral fibrosis was seen in a slight to moderate degree. There was a moderate to severe infiltrating growth, sometimes with invasion of lymph- and blood vessels. The intratumoral necrosis and/or cyst formation ranged from 5% to 40% of the total tumor area. The tumor cells revealed a moderate to severe rate of proliferation (mitosis per field of view by 200x magnification) and of apoptosis (apoptotic cells per field of a view by 200x magnification). A moderate to high number of infiltrating inflammatory cells (neutrophils, lymphocytes and macrophages) was present. For photographic pictures see Figure 1.

**Table 2: Histopathological analysis of primary tumors -Group 1.**

| **Animal no.** | **Cell. + nucl. Polymorphism** | **Rate of proliferation (mitosis per field of view)** | **Necrosis / Cyst formation % of total area** | **Apoptosis (per field of view)** | **Invasive growth** | **Capsule formation** | **Intratumoral fibrosis** | **Inflammatory cell infiltration** |
|---|---|---|---|---|---|---|---|---|
| 101 | ++ -+++ | ++ | 5 | ++ -+++ | ++ | + | + -++ | + -++ |
| 102 | ++ -+++ | ++ -+++ | 5 | ++ -+++ | +++ | + | + -++ | +++ |
| 103 | ++ -+++ | ++ | 10 | ++ -+++ | ++ | + -++ | + -++ | ++ |
| 104 | ++ -+++ | ++ | 10 | +++ | +++ | + -++ | + -++ | ++ -+++ |
| 105 | ++ -+++ | ++ | 40 | +++ | +++ | + -++ | + -++ | ++ -+++ |
| 106 | ++ -+++ | ++ | 20 | +++ | ++ | + -++ | + -++ | + -++ |
| 107 | ++ -+++ | ++ -+++ | 20 | +++ | +++ | + -++ | + -++ | + -++ |
| 108 | ++ -+++ | ++ -+++ | 10 | +++ | ++ | + -++ | + -++ | ++ -+++ |
| 109 | ++ -+++ | ++ -+++ | 5 | +++ | ++ | + -++ | + -++ | ++ |
| 110 | ++ -+++ | ++ -+++ | 20 | +++ | ++ | + -++ | + -++ | ++ -+++ |

| | | | | | | | | |
|---|---|---|---|---|---|---|---|---|
| (+) = minimal + = slight ++ = moderate +++ severe ++++ = extreme | | | | | | | | |

### Group 2: Erlotinib HCl, 50 mg/kg

In this group the tumors were distinctly smaller in size than the tumors in the control group and revealed a strong loss of tumor cells with a distinct increase of intratumoral fibrosis and capsule formation. Compared to the controls, the rate of proliferation was strongly decreased, invasive growth and apoptosis was moderately reduced. The tumor cells showed a more uniform appearance. Intratumoral necrosis was not present. For photographic pictures see Fig 1.

**Table 3: Histopathological analysis of primary tumors -Group 2.**

| **Animal no.** | **Cell. + nucl. Polymorphism** | **Rate of proliferation (mitosis per field of view)** | **Necrosis / Cyst formation % of total area** | **Apoptosis (per field of view)** | **Invasive growth** | **Capsule formation** | **Intratumoral fibrosis** | **Inflam matory cell infiltration** |
|---|---|---|---|---|---|---|---|---|
| 201* | ++ | (+) | 0 | ++ | + | ++ | +++ | + |
| 202* | ++ | (+) | 0 | ++ | + | ++ | +++ | ++ |
| 203 | ++ | (+) | 0 | + -++ | ++ | ++ | ++ -+++ | ++ |
| 204* | ++ | (+) | 0 | ++ -+++ | ++ | + -++ | ++ -+++ | ++ -+++ |
| 205 | ++ | (+) | 0 | ++ | + -++ | ++ | ++ -+++ | ++ |
| 206* | ++ | (+) | 0 | + -++ | + | ++ | ++ -+++ | + |
| 207* | ++ | (+) | 0 | + -++ | + -++ | ++ | ++ -+++ | ++ |
| 208* | ++ | (+) | 0 | + -++ | + | ++ | +++ | + -++ |
| 209* | ++ | (+) | 0 | + -++ | + | ++ | +++ | ++ -+++ |
| 210* | ++ | (+) | 0 | + -++ | + | ++ | +++ | ++ -+++ |

| | | | | | | | | |
|---|---|---|---|---|---|---|---|---|
| (+) = minimal + = slight ++ = moderate +++ severe ++++ = extreme * = loss of tumor cell compared to control | | | | | | | | |

### Group 3: <IGF-1R> HUMAB Clone 18 - LD 36 mg/kg, MD 18 mg/kg

In this group there was a slight to moderate increase of capsule formation and intratumoral fibrosis with a loss of tumor cells compared to the control (group 1). The tumors were smaller in size as the tumors of the control group. Invasive growth and the rate of proliferation and apoptosis was slightly reduced compared to the control. There was a distinct decrease of intratumoral necrosis in these tumors. For photographic pictures see Fig 1.

**Table 4: Histopathological analysis of primary tumors -Group 3.**

| **Ani mal no.** | **Cell. + nucl. Polymorphism** | **Rate of proliferation (mitosis per field of view)** | **Necrosis / Cyst formation % of total area** | **Apoptosis (per field of view)** | **Invasive growth** | **Capsule formation** | **Intratumoral fibrosis** | **Inflammatory cell infiltration** |
|---|---|---|---|---|---|---|---|---|
| 301* | ++ -+++++ | ++ | 0 | ++ -+++ | + | ++ | ++ | +++ |
| 302 | ++ -+++ | ++ -+++ | 0 | +++ | ++ | + | + -++ | +++ |
| 303* | ++ | +-++ | 0 | ++ | + | ++ | +++ | ++ |
| 304 | ++ -+++ | ++ -+++ | 5 | ++ -+++ | ++ -+++ | + -++ | + -++ | +++ |
| 306* | + -++ | (+) | 0 | ++ -+++ | + | ++ -+++ | +++ | + |
| 307* | + -++ | (+) | 0 | ++ -+++ | + | ++ | +++ | + |
| 308 | ++ -+++ | + -++ | 0 | ++ | ++ | ++ | ++ -+++ | ++ |
| 309* | ++ | + -++ | 0 | ++ | + | ++ -+++ | +++ | + |
| 310 | ++ -+++ | + -++ | 5 | ++ | +++ | ++ | ++ | +++ |
| 311 | ++ -+++ | ++ | 5 | ++ | ++ | + -++ | + -++ | +++ |

| | | | | | | | | |
|---|---|---|---|---|---|---|---|---|
| (+) = minimal + = slight ++ = moderate +++ severe ++++ = extreme * = loss of tumor cell compared to control | | | | | | | | |

### Group 4: Erlotinib 50 mg/kg in combination with <IGF-1R> HUMAB Clone 18 -LD 36 mg/kg, MD 18 mg/kg

Compared to the tumors in groups 1, 2 and 3, the tumors of this group were very small in size and revealed a very strong loss of tumor cells with an extreme increase of intratumoral fibrosis and capsule formation. The residual tumor cells showed an uniform appearance. Intratumoral necrosis and invasive growth was not present. There were no signs of proliferating tumor cells (mitotic figures = 0). Apoptosis and inflammatory cell infiltration was strongly decreased compared to the control tumors (group 1). For photographic pictures see Fig 1.

**Table 5: Histopathological analysis of primary tumors -Group 4.**

| **Animal no.** | **Cell. + nucl. Polymorphism** | **Rate of proliferation (mitosis per field of view)** | **Necrosis / Cyst formation % of total area** | **Apoptosis (per field of view)** | **Invasive growth** | **Capsule formation** | **Intratumoral fibrosis** | **Inflammatory cell infiltration** |
|---|---|---|---|---|---|---|---|---|
| 401** | + | neg | 0 | + | - | +++ | ++++ | (+) |
| 402** | + | neg | 0 | +-++ | - | +++ | ++++ | (+) |
| 403** | + | neg | 0 | +-++ | - | +++ | ++++ | (+) |
| 404** | + | neg | 0 | + | - | +++ | ++++ | (+) |
| 405** | + | neg | 0 | + | - | +++ | ++++ | (+) |
| 406** | + | neg | 0 | ++ | - | +++ | ++++ | + |
| 407** | + | neg | 0 | + -++ | - | +++ | ++++ | + -++ |
| 408** | + | neg | 0 | + -++ | - | +++ | ++++ | (+) |
| 409** | + | neg | 0 | + -++ | - | +++ | ++++ | (+) |
| 410** | + | neg | 0 | + -++ | - | +++ | ++++ | (+) |

| | | | | | | | | |
|---|---|---|---|---|---|---|---|---|
| (+) = minimal + = slight ++ = moderate +++ severe ++++ = extreme ** extreme loss of tumor cells - no signs of invasive growth | | | | | | | | |

In this study, mice engrafted with H322M (human NSCLC cell line) were treated under single agent therapy either with 50 mg/kg erlotinib (Tarceva; group 2) or with 36 mg/kg (LD) and 18 mg/kg (MD) <IGF-1R> HUMAB Clone 18 (huMab IGF-1R; group 3). Animals of group 4 were treated with both agents in combination. The histopathological investigations showed the following results: Compared to the control (group 1) treatment with erlotinib (Tarceva) and <IGF-1R> HUMAB Clone 18 as single agent treatment revealed distinct drug related histopathological findings. There was a decrease of tumor size connected with an increase of intratumoral fibrosis, capsule formation and a loss of tumor cells. There was a decrease in the rate of proliferation, apoptosis and intratumoral necrosis. The invasive growth of these tumors were reduced. All these changes were more pronounced in tumors treated solely with erlotinib (Tarceva) compared to single agent treatment with <IGF-1R> HUMAB Clone 18. Additionally, tumors treated with erlotinib (Tarceva) showed a distinct reduction of the tumor cell polymorphism (uniform tumor cells versus polymorph tumor cells in controls).

The combination treatment with erlotinib (Tarceva) and <IGF-1R> HUMAB Clone 18 showed considerable histopathological changes compared to the control group (group 1) and the single agent treatment groups (group 2 and 3): The tumor size was strongly reduced with a very strong loss of tumor cells connected with an extreme increase of intratumoral fibrosis and capsule formation. The residual tumor cells in the fibrous tissue showed an uniform appearance with absence of invasive growth or proliferative activity (no mitotic figures were present). The apoptotic index was strongly reduced compared to the control and the single agent groups. Intratumoral necrosis was not present.

Combination treatment with erlotinib (Tarceva) and <IGF-1R> HUMAB Clone 18 showed synergistic histopathological effects compared to single agent treatment and led to a strong growth regression of H322M xenograft tumors. Due to the extreme increase of intratumoral fibrosis, filling up the space left by lost tumor cells, the histological investigation in this case is the more valid evaluation method compared to measurement of tumor weight or tumor volume.

The tumors of group 4 consisted mostly of fibrous tissue, mixed with a few nests of not proliferating and uniform tumor cells. Thus, the real reduction in tumor mass (amount of viable and proliferating tumor cells) cannot correctly be shown by caliper measurements. The superior antitumoral effect of the erlotinib (Tarceva) and <IGF-1R> HUMAB Clone 18 combination treatment may give an argument for additional combination studies with <IGF-1R> HUMAB Clone 18, Tarceva and/or other target specific drugs in xenograft tumors expressing more than one target.

## Claims

1. Use of an antibody binding to human IGF-1R comprising as complementarity determining regions (CDRs) the following sequences:
a) in the heavy chain variable domain a CDR3 region of SEQ ID NO: 1, a CDR2 region of SEQ ID NO: 2, and a CDR1 region of SEQ ID NO:3, and in the light chain variable domain a CDR3 region of SEQ ID NO: 4, a CDR2 region of SEQ ID NO:5, and a CDR1 region of SEQ ID NO:6; or
b) in the heavy chain variable domain a CDR3 region of SEQ ID NO: 7, a CDR2 region of SEQ ID NO: 8, and a CDR1 region of SEQ ID NO:9, and in the light chain variable domain a CDR3 region of SEQ ID NO: 10, a CDR2 region of SEQ ID NO:11, and a CDR1 region of SEQ ID NO:12;
for the manufacture of a medicament for treatment of an IGF-1R and EGFR expressing cancer **characterized in that** said antibody is co-administered with erlotinib.

2. The use according to claim 1, wherein the antibody binding to human IGF-1 R comprises the following sequences:
a) as heavy chain variable domain SEQ ID NO: 13 or SEQ ID NO: 14, and
b) as light chain variable domain a SEQ ID NO: 15 or SEQ ID NO: 16.

3. The use according to claim 1, wherein the antibody binding to human IGF-1 R comprises the following sequences:
a) as heavy chain variable domain SEQ ID NO: 13, and
b) as light chain variable domain a SEQ ID NO: 15.

4. The use according to claim 1, wherein the antibody binding to human IGF-1 R comprises the following sequences:
a) as heavy chain variable domain SEQ ID NO: 14, and
b) as light chain variable domain a SEQ ID NO: 16.

5. The use according to any one of claims 1 to 4, wherein the IGF-1R and EGFR expressing cancer is a lung cancer.

6. The use according to any one of claims 1 to 4, wherein the IGF-1R and EGFR expressing cancer is a non small cell lung (NSCL) cancer.

7. An antibody binding to human IGF-1R comprising as complementarity determining regions (CDRs) the following sequences:
a) in the heavy chain variable domain a CDR3 region of SEQ ID NO: 1, a CDR2 region of SEQ ID NO: 2, and a CDR1 region of SEQ ID NO:3, and in the light chain variable domain a CDR3 region of SEQ ID NO: 4, a CDR2 region of SEQ ID NO:5, and a CDR1 region of SEQ ID NO:6; or
b) in the heavy chain variable domain a CDR3 region of SEQ ID NO: 7, a CDR2 region of SEQ ID NO: 8, and a CDR1 region of SEQ ID NO:9, and in the light chain variable domain a CDR3 region of SEQ ID NO: 10, a CDR2 region of SEQ ID NO:11, and a CDR1 region of SEQ ID NO:12; for the treatment of cancer for treatment of cancer **characterized in that** said antibody is co-administered with erlotinib

8. A kit comprising
i) an antibody binding to human IGF-1R comprising as complementarity determining regions (CDRs) the following sequences:
a) in the heavy chain variable domain a CDR3 region of SEQ ID NO: 1, a CDR2 region of SEQ ID NO: 2, and a CDR1 region of SEQ ID NO:3, and in the light chain variable domain a CDR3 region of SEQ ID NO: 4, a CDR2 region of SEQ ID NO:5, and a CDR1 region of SEQ ID NO:6; or
b) in the heavy chain variable domain a CDR3 region of SEQ ID NO: 7, a CDR2 region of SEQ ID NO: 8, and a CDR1 region of SEQ ID NO:9, and in the light chain variable domain a CDR3 region of SEQ ID NO: 10, a CDR2 region of SEQ ID NO:11, and a CDR1 region of SEQ ID NO:12; and
ii) erlotinib
for the combination treatment of a patient suffering from a IGF-1R and EGFR expressing cancer.

9. A pharmaceutical composition comprising both
i) an antibody binding to human IGF-1R comprising as complementarity determining regions (CDRs) the following sequences:
a) an antibody heavy chain comprising as CDRs CDR1 (aa 31-35), CDR2 a) in the heavy chain variable domain a CDR3 region of SEQ ID NO: 1, a CDR2 region of SEQ ID NO: 2, and a CDR1 region of SEQ ID NO:3, and in the light chain variable domain a CDR3 region of SEQ ID NO: 4, a CDR2 region of SEQ ID NO:5, and a CDR1 region of SEQ ID NO:6; or
b) in the heavy chain variable domain a CDR3 region of SEQ ID NO: 7, a CDR2 region of SEQ ID NO: 8, and a CDR1 region of SEQ ID NO:9, and in the light chain variable domain a CDR3 region of SEQ ID NO: 10, a CDR2 region of SEQ ID NO:11, and a CDR1 region of SEQ ID NO:12; and
ii) erlotinib
for use in the treatment of an IGF-1R and EGFR expressing cancer, wherein said pharmaceutical composition may further comprise one or more pharmaceutically acceptable carriers.


SEQUENCE LISTING

<110>  F. Hoffmann-La Roche

<120>  Combination therapy of erlotinib with an anti-IGF-1R antibody,
       which does not inhibit binding of insulin to the insulin receptor

<130>  Case 26055

<160>  21

<170>  PatentIn version 3.2

<210>  1
<211>  9
<212>  PRT
<213>  Artificial

<220>
<223>  heavy chain CDR3, <IGF-1R> HUMAB-Clone 18

<400>  1

Glu Leu Gly Arg Arg Tyr Phe Asp Leu
1               5

<210>  2
<211>  17
<212>  PRT
<213>  Artificial

<220>
<223>  heavy chain CDR2, <IGF-1R> HUMAB-Clone 18

<400>  2

Ile Ile Trp Phe Asp Gly Ser Ser Thr Tyr Tyr Ala Asp Ser Val Arg
1               5                   10                  15

Gly

<210>  3
<211>  5
<212>  PRT
<213>  Artificial

<220>
<223>  heavy chain CDR1, <IGF-1R> HUMAB-Clone 18

<400>  3

Ser Tyr Gly Met His
1               5


<210>  4
<211>  10
<212>  PRT
<213>  Artificial

<220>
<223>  light chain CDR3, <IGF-1R> HUMAB-Clone 18

<400>  4

Gln Gln Arg Ser Lys Trp Pro Pro Trp Thr
1               5                   10


<210>  5
<211>  7
<212>  PRT
<213>  Artificial

<220>
<223>  light chain CDR2, <IGF-1R> HUMAB-Clone 18

<400>  5

Asp Ala Ser Lys Arg Ala Thr
1               5


<210>  6
<211>  11
<212>  PRT
<213>  Artificial

<220>
<223>  light chain CDR1, <IGF-1R> HUMAB-Clone 18

<400>  6

Arg Ala Ser Gln Ser Val Ser Ser Tyr Leu Ala
1               5                   10


<210>  7
<211>  9
<212>  PRT
<213>  Artificial

<220>
<223>  heavy chain CDR3, <IGF-1R> HUMAB-Clone 22

<400>  7

Glu Leu Gly Arg Arg Tyr Phe Asp Leu
1               5

<210>  8
<211>  17
<212>  PRT
<213>  Artificial

<220>
<223>  heavy chain CDR2, <IGF-1R> HUMAB-Clone 22

<400>  8

Ile Ile Trp Phe Asp Gly Ser Ser Lys Tyr Tyr Gly Asp Ser Val Lys
1               5                   10                  15

Gly

<210>  9
<211>  5
<212>  PRT
<213>  Artificial

<220>
<223>  heavy chain CDR1, <IGF-1R> HUMAB-Clone 22

<400>  9

Ser Tyr Gly Met His
1               5

<210>  10
<211>  10
<212>  PRT
<213>  Artificial

<220>
<223>  light chain CDR3, <IGF-1R> HUMAB-Clone 22

<400>  10

Gln Gln Arg Ser Lys Trp Pro Pro Trp Thr
1               5                   10

<210>  11
<211>  7
<212>  PRT

<213>  Artificial

<220>
<223>  light chain CDR2, <IGF-1R> HUMAB-Clone 22

<400>  11

Asp Ala Ser Asn Arg Ala Thr
1               5


<210>  12
<211>  11
<212>  PRT
<213>  Artificial

<220>
<223>  light chain CDR1, <IGF-1R> HUMAB-Clone 22

<400>  12

Arg Ala Ser Gln Ser Val Ser Ser Tyr Leu Ala
1               5                   10


<210>  13
<211>  118
<212>  PRT
<213>  Homo sapiens

<400>  13

Gln Val Glu Leu Val Glu Ser Gly Gly Gly Val Val Gln Pro Gly Arg
1               5                   10                  15


Ser Gln Arg Leu Ser Cys Ala Ala Ser Gly Phe Thr Phe Ser Ser Tyr
            20                  25                  30


Gly Met His Trp Val Arg Gln Ala Pro Gly Lys Gly Leu Glu Trp Val
        35                  40                  45


Ala Ile Ile Trp Phe Asp Gly Ser Ser Thr Tyr Tyr Ala Asp Ser Val
    50                  55                  60


Arg Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Phe Cys
                85                  90                  95

Ala Arg Glu Leu Gly Arg Arg Tyr Phe Asp Leu Trp Gly Arg Gly Thr
            100                 105                 110

Leu Val Ser Val Ser Ser
        115

<210>  14
<211>  117
<212>  PRT
<213>  Homo sapiens

<400>  14

Val Gln Leu Val Glu Ser Gly Gly Gly Val Val Gln Pro Gly Arg Ser
1               5                   10                  15

Leu Arg Leu Ser Cys Ala Ala Ser Gly Phe Thr Phe Ser Ser Tyr Gly
            20                  25                  30

Met His Trp Val Arg Gln Ala Pro Gly Lys Gly Leu Glu Trp Met Ala
        35                  40                  45

Ile Ile Trp Phe Asp Gly Ser Ser Lys Tyr Tyr Gly Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Asp Asp Thr Ala Val Tyr Tyr Cys Ala
                85                  90                  95

Arg Glu Leu Gly Arg Arg Tyr Phe Asp Leu Trp Gly Arg Gly Thr Leu
            100                 105                 110

Val Thr Val Ser Ser
        115

<210>  15
<211>  108
<212>  PRT
<213>  Homo sapiens

<400>  15

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Lys Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Lys Trp Pro Pro
                85                  90                  95

Trp Thr Phe Gly Gln Gly Thr Lys Val Glu Ser Lys
            100                 105

<210>  16
<211>  108
<212>  PRT
<213>  Homo sapiens

<400>  16

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Lys Trp Pro Pro
                85                  90                  95

Trp Thr Phe Gly Gln Gly Thr Lys Val Glu Ile Lys
            100                 105

<210>  17
<211>  330
<212>  PRT
<213>  Homo sapiens

<400>  17

<210>  18
<211>  327
<212>  PRT
<213>  Homo sapiens

<400>  18

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210> 19
<211> 107
<212> PRT
<213> Homo sapiens

<400> 19

<210>  20
<211>  1367
<212>  PRT
<213>  Homo sapiens

<400>  20

<210> 21
<211> 1210
<212> PRT
<213> Homo sapiens

<400> 21


1               5                   10                  15


            20                  25                  30


        35                  40                  45


    50                  55                  60


65                  70                  75                  80


                85                  90                  95


            100                 105                 110


        115                 120                 125


    130                 135                 140


145                 150                 155                 160


                165                 170                 175


            180                 185                 190


        195                 200                 205


    210                 215                 220


225                 230                 235                 240


                245                 250                 255


            260                 265                 270


        275                 280                 285



290                 295                 300


305                 310                 315                 320


                325                 330                 335


            340                 345                 350


        355                 360                 365


    370                 375                 380


385                 390                 395                 400


                405                 410                 415


            420                 425                 430


        435                 440                 445


    450                 455                 460


465                 470                 475                 480


                485                 490                 495


            500                 505                 510


515 520 525


530 535 540


545 550 555 560


565 570 575


580 585 590


595 600 605


610 615 620


625 630 635 640


645 650 655


660 665 670


675 680 685


690 695 700


705 710 715 720


725 730 735


740 745 750


755 760 765


770 775 780


785 790 795 800


805 810 815


820 825 830


835 840 845


850 855 860


865 870 875 880


885 890 895


900 905 910


915 920 925


930 935 940


945 950 955 960


965 970 975


980 985 990


995 1000 1005


1010 1015 1020


1025 1030 1035


1040 1045 1050


1055 1060 1065


1070 1075 1080


1085 1090 1095


1100 1105 1110


1115 1120 1125


1130 1135 1140


1145 1150 1155



1160                1165                1170


    1175                1180                1185


    1190                1195                1200


    1205                1210